# EUROPÄISCHE PATENTSCHRIFT

(11) **EP 2 218 415 B1**
(45) Veröffentlichungstag und Bekanntmachung des Hinweises auf die Patenterteilung: **04.05.2011**
(21) Anmeldenummer: 09002104.9
(22) Anmeldetag: 16.02.2009
(51) Int. Cl.: A61B 17/86

(54) **Knochenschraube und Herstellungsverfahren hierfür**
Bone screw and production method therefor
Vis à os et procédé de fabrication correspondant

(43) Veröffentlichungstag der Anmeldung: 18.08.2010
(73) Patentinhaber: Stryker Trauma AG, 2545 Selzach (CH)
(72) Erfinder: Christen, Alexis, 3006 Bern (CH); Markovic, Zeljko, 2545 Selzach (CH)
(74) Vertreter: Röthinger, Rainer

(56) Entgegenhaltungen:
- WO-A-2007/048267
- US-A- 6 030 162
- US-A1- 2006 195 099
- US-A1- 2008 188 899

## Beschreibung

### Technisches Gebiet

Die Erfindung betrifft eine selbstschneidende Knochenschraube, insbesondere zur Anwendung als Verriegelungsschraube für ein Implantat. Die Erfindung betrifft ferner ein Herstellungsverfahren für eine solche Knochenschraube.

### Technischer Hintergrund

Knochenschrauben sind Schrauben, die in Knochen eingedreht werden. Grundsätzlich werden Knochenschrauben auf zwei verschiedene Arten verwendet: Bei einer ersten Anwendung dienen Knochenschrauben dazu, Knochen oder Knochenfragmente in einer gewünschten Position zueinander zu fixieren. Hierbei wird die Knochenschraube alleine verwendet. Bei einer zweiten Anwendung wird die Knochenschraube als Verriegelungsschraube benutzt, um zusätzliche Elemente als Fixationselemente im oder am Knochen zu positionieren. Knochenschrauben werden hier beispielsweise zusammen mit Marknägeln eingesetzt. Ein anderes Anwendungsgebiet ist die Osteosynthese, bei der ein biokompatibles Element einen Knochen oder ein Knochenfragment substituiert. Beispielsweise kann eine aus Titan bestehende Platte als Ersatz eines Schädelfragmentes durch Knochenschrauben am Schädel verankert werden.

Für spezielle Anwendungen sind Knochenschrauben in einer Vielzahl von Variationen verfügbar. So offenbart beispielsweise die US 6,030,162 eine Knochenschraube zur Erzeugung einer axialen Kompression, so dass Knochenfragmente durch die eingedrehte Schraube zusammengepresst werden. Die Kompression wird unter anderem durch das Vorsehen mehrerer Gewindeabschnitte mit unterschiedlichen Gewindesteigungen erzeugt.

In vielen Fällen ist der Schraubenschaft einer Knochenschraube zylindrisch ausgebildet. Aus der EP 0 491 211 A1 ist eine Knochenschraube bekannt, welche über einen kopfseitigen, zylindrischen ersten Schaftabschnitt und über einen spitzenseitigen, an den ersten Schaftabschnitt anschließenden, zweiten und ebenfalls zylindrischen Schaftabschnitt verfügt, wobei der erste Schaftabschnitt einen größeren Kerndurchmesser aufweist als der zweite Schaftabschnitt. In wieder anderen Fällen haben Knochenschrauben einen sich von der Spitze zum Kopf hin verbreiternden, konisch geformten Schraubenschaft.

Aus der WO 2007/048267 A1 ist eine Knochenschraube bekannt, bei der ein Kerndurchmesser in einem an der Spitze der Schraube gelegenem Vorformbereich größer ist als der Kerndurchmesser in einem an den Vorformbereich anschließenden Zwischenbereich. Ein Außendurchmesser der Schraube im Vorformbereich ist ebenfalls größer als ein Außendurchmesser im Zwischenbereich.

Selbstschneidende Schrauben haben den Vorteil, dass im Knochen kein Gewinde vorgeschnitten werden muss. Derartige Schrauben verfügen über einen Schraubenschaft mit mindestens einem Gewindeabschnitt. Das Gewinde ist in Bezug auf Eigenschaften wie Gewindeprofil, Flankenwinkel, etc. geeignet ausgebildet, so dass sich die Schraube ihr Gewinde selbst schneidet, wenn sie der Chirurg in das Knochenmaterial eindreht.

Ein grundsätzliches Problem bei selbstschneidenden Knochenschrauben stellen die zum Teil erheblichen Eindrehkräfte dar, die beim Eindrehen der Schraube in den Knochen auftreten. Das Material eines Knochens verhält sich beim Durchschneiden in gewissem Maße elastisch; das heißt, dass nach dem Durchschneiden das Knochenmaterial in seine Ausgangslage zurückstrebt. Dies erhöht die durch den Chirurgen aufzubringende Kraft, und zwar bei zunehmender Eindrehtiefe in erheblichem Umfang. Die Problematik wird noch zusätzlich verschärft, wenn der Chirurg in einem kleinen Gebiet operiert, etwa im Gesichts- oder Schädelbereich.

### Kurzer Abriss

Der Erfindung liegt die Aufgabe zugrunde, eine selbstschneidende Knochenschraube vorzuschlagen, bei der die Eindrehkräfte reduziert sind ohne dass der sichere und passgenaue Sitz der Schraube und damit ihre Funktion nachteilig beeinflusst werden.

Um diese Aufgabe zu lösen, wird gemäß einem ersten Aspekt eine selbstschneidende Knochenschraube vorgeschlagen. Die Knochenschraube hat einen Schraubenschaft, der eine vordere Spitze, einen Schneidebereich, einen Zwischenbereich und einen hinteren Kopfbereich aufweist. In einem Gewindeabschnitt des Schraubenschaftes erstreckt sich ein Gewinde über einen Übergangsbereich, der aneinander angrenzende Teile wenigstens des Schneidebereichs und des Zwischenbereichs umfasst. Durch das Gewinde sind im Gewindebereich ein Außendurchmesser und ein Kerndurchmesser des Schraubenschaftes definiert. Im Übergangsbereich ist der Kerndurchmesser des Schraubenschaftes im Schneidebereich größer als der Kerndurchmesser des Schraubenschaftes im Zwischenbereich. Weiterhin ist im Übergangsbereich der Außendurchmesser des Schraubenschaftes konstant.

Der Kerndurchmesser kann am Übergang vom Schneidebereich zum Zwischenbereich abgestuft sein, beispielsweise in Form einer einzelnen Stufe oder mehrerer Stufen. Bei einigen Varianten der vorgeschlagenen Knochenschraube hat der Kern des Schneidebereichs eine konvexe Form. Bei anderen Varianten ist der Kern des Schneidebereichs abgestuft. Auch Mischformen aus konvexer und abgestufter Kontur sind denkbar. Unabhängig davon kann der Kerndurchmesser des Schraubenschaftes im Zwischenbereich konstant sein oder aber variieren. Der Außendurchmesser des Schraubenschaftes im Gewindeabschnitt kann im Zwischenbereich konstant sein. Bei einigen Ausführungen der Knochenschraube erstreckt sich das Gewinde durchgehend über den Schneidebereich und den Zwischenbereich (und u.U. auch bis in den Kopfbereich). Das Gewinde kann eine konstante Gewindesteigung haben.

Das Gewinde kann im Schneidebereich zumindest bereichsweise als Trapezgewinde ausgeführt sein. Im Zwischenbereich kann das Gewinde als Spitzgewinde dargestellt sein. Bei manchen Ausführungen der Knochenschraube läuft das Gewinde zur Spitze hin aus. Hiervon unabhängig kann die Spitze als Zentrierspitze ausgeführt sein. Beispielsweise kann die Spitze gestuft oder abgerundet ausgeführt sein.

Gemäß einer Variante der Knochenschraube verfügt diese über eine sich wenigstens über die Spitze und den Schneidebereich erstreckende Nut zum Wegführen von Schnittgut. Auch zwei, drei oder mehr solcher Nuten können vorgesehen werden.

Der Kopfbereich der Knochenschraube kann ein Gewinde aufweisen. Bei anderen Realisierungen ist der Kopfbereich gewindefrei. Der Außendurchmesser des Kopfbereiches kann größer sein als der Außendurchmesser des Zwischenbereiches. Auch kann der Kerndurchmesser des Kopfbereiches größer sein als der Kerndurchmesser des Zwischenbereiches.

Gemäß einer Alternative verfügt die Knochenschraube über ein von der Spitze bis zum Kopfbereich durchgehendes Gewinde. In einer Variante dieser Ausführung weist der Kopfbereich einen jeweils gegenüber dem Zwischenbereich vergrößerten Kern- bzw. Außendurchmesser auf. Bei einer anderen Variante sind Kern- und Außendurchmesser im Kopfbereich und Zwischenbereich konstant.

In einer Variante weist die Knochenschraube einen spitzenseitigen Gewindeabschnitt und einen kopfseitigen Gewindeabschnitt auf. Die Gewindeabschnitte sind durch einen gewindefreien Teil des Zwischenbereichs voneinander getrennt. Die Gewinde in den beiden Gewindeabschnitten können zueinander synchron verlaufen. Der Kopfbereich mit dem kopfseitigen Gewindeabschnitt kann einen größeren Kern- und Außendurchmesser als der Zwischenbereich haben.

Eine Realisierung der hier angerissenen Knochenschraube ist zur Anwendung als Verriegelungsschraube für ein Implantat bestimmt.

Weiterhin wird gemäß einem weiteren Aspekt ein Verfahren zur Herstellung einer selbstschneidenden Knochenschraube vorgeschlagen. Die Knochenschraube hat einen Schraubenschaft, der eine vordere Spitze, einen Schneidebereich, einen Zwischenbereich und einen hinteren Kopfbereich aufweist. In einem Gewindeabschnitt des Schraubenschaftes erstreckt sich ein Gewinde über einen Übergangsbereich, der aneinander angrenzende Teile wenigstens des Schneidebereichs und des Zwischenbereichs umfasst. Durch das Gewinde sind im Gewindebereich ein Außendurchmesser und ein Kerndurchmesser des Schraubenschaftes definiert. Das Verfahren umfasst den Schritt, ein Fräswerkzeug zur Herstellung des Gewindes im Gewindeabschnitt derart zu führen, dass die Gewindezähne im Schneidebereich weniger tief geschnitten werden als im Zwischenbereich. Auf diese Weise ist im Übergangsbereich der Kerndurchmesser des Schraubenschaftes im Schneidebereich größer als der Kerndurchmesser des Schraubenschaftes im Zwischenbereich. Außerdem ist der Außendurchmesser des Schraubenschaftes konstant.

### Kurzbeschreibung der Zeichnungen

Weitere Aspekte und Vorteile der Erfindung ergeben sich aus der nachfolgenden Beschreibung bevorzugter Ausführungsbeispiele sowie aus den Figuren. Es zeigen:
- Fig. 1: eine Seitenansicht eines ersten Ausführungsbeispiels einer Knochen- schraube;
- Fig. 2: eine Seitenansicht der um 90° gedrehten Knochenschraube aus Fig. 1;
- Fig. 3: eine vergrößerte Seitenansicht eines vorderen Abschnitts der Knochen- schraube aus Fig. 1;
- Fig. 4: eine vergrößerte Seitenansicht eines vorderen Abschnitts der Knochen- schraube aus Fig. 2;
- Fig. 5: eine Aufsicht auf die Spitze der Knochenschraube aus Fig. 1;
- Fig. 6: eine Seitenansicht eines vorderen Abschnitts eines Schraubenrohlings;
- Fig. 7: einen Schnitt durch einen vorderen Abschnitt der Knochenschraube aus Fig. 1;
- Fig. 8: ein zweites Ausführungsbeispiel einer Knochenschraube mit einem gegenüber der Schraube aus Fig. 1 veränderten Kopfbereich;
- Fig. 9: ein drittes Ausführungsbeispiel einer Knochenschraube mit einem ge- genüber den Schrauben aus den Figuren 1 und 8 veränderten Kopfbe- reich;
- Fig. 10: ein viertes Ausführungsbeispiel einer Knochenschraube mit einem ge- genüber den vorherigen Beispielen nochmals veränderten Kopfbereich;
- Fig. 11: eine Aufsicht auf die Spitze der Knochenschraube aus Fig. 10;
- Fig. 12: einen Schnitt durch den Schneidebereich der Knochenschraube aus Fig. 10;
- Fig. 13: eine Schnittansicht eines hinteren Teils der Knochenschraube aus Fig. 10; und
- Fig. 14: eine Aufsicht auf den Kopfbereich der Knochenschraube aus Fig. 10.

### Detaillierte Beschreibung

Nachfolgend werden einige Ausführungsbeispiele einer Knochenschraube erläutert. Bei verschiedenen Ansichten ein und desselben Ausführungsbeispiels werden für identische Elemente dieselben Bezugszeichen verwendet.

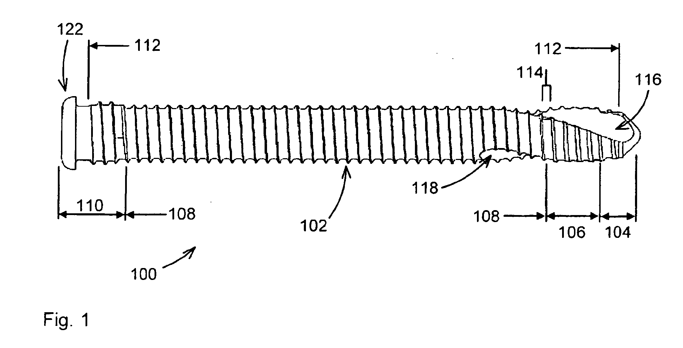

Anhand der Fign. 1-7 wird zunächst ein erstes Ausführungsbeispiel einer Knochenschraube 100 erläutert, die beispielsweise als Verriegelungsschraube für den Einsatz in der Osteosynthese im Gesichts-/Schädelbereich vorgesehen sein kann. Fig. 1 zeigt eine Seitenansicht der Knochenschraube 100, die als selbstschneidende Schraube ausgeführt ist. Die Knochenschraube 100 hat einen Schraubenschaft 102 mit einer vorderen Spitze 104, einem Schneidebereich 106, einem Zwischenbereich 108 und einem Kopfbereich 110. Ein Gewinde erstreckt sich über einen Gewindeabschnitt 112, der sich bei diesem Ausführungsbeispiel durchgehend von der Spitze 104 bis in den Kopfbereich 110 hinein erstreckt. Im vorderen Bereich des Schraubenschaftes 102 sind zwei helixartig gewundene Nuten 116 und 118 zum Wegführen von Schnittgut vorgesehen.

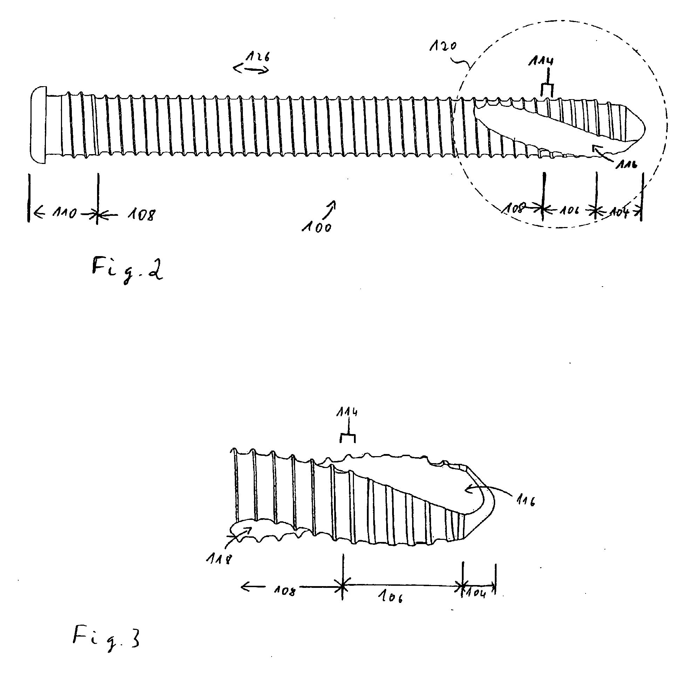

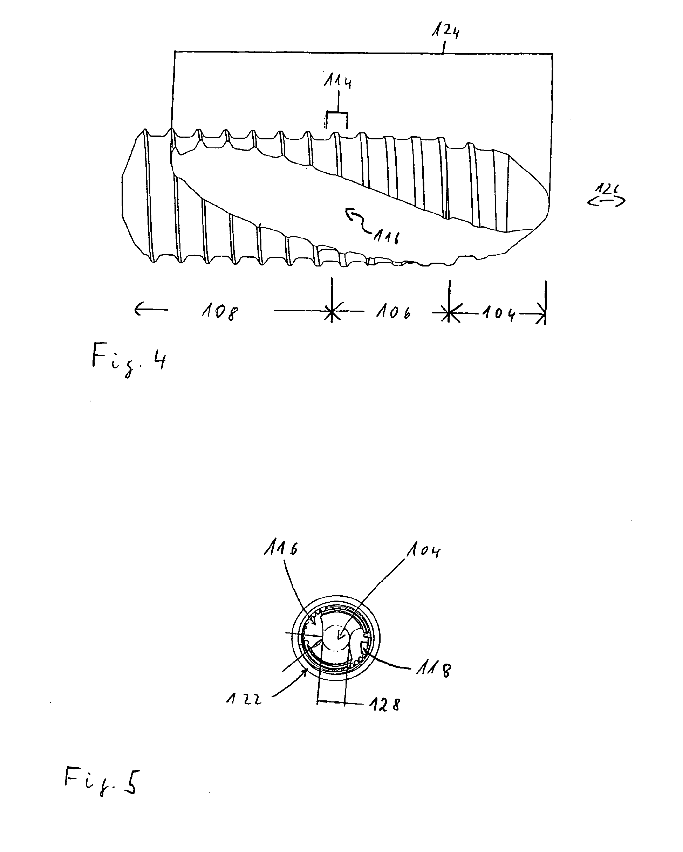

Fig. 2 zeigt die Knochenschraube 100 in einer um eine Vierteldrehung um die Längsachse rotierten Ansicht. Ein Ausschnitt der Seitenansichten der Schraube 100 aus den Fign. 1 und 2 (s. in Fig. 2 den durch den Kreis 120 bezeichneten Ausschnitt) ist in den Fign. 3 bzw. 4 vergrößert dargestellt. Es ist beispielsweise aus Fig. 4 erkennbar, dass sich die Nut 116 über die Spitze 104 und den Schneidebereich 106 in den Zwischenbereich 108 hinein erstreckt und dort ausläuft. Statt zwei Nuten kann auch nur eine Nut oder mehrere Nuten, beispielsweise 3 oder 4 Nuten vorgesehen sein. Fig. 5 zeigt eine Ansicht von vorne auf die Spitze 104 der Schraube 100. Erkennbar sind die Nuten 116 und 118 sowie der Kopfteil 122 (vgl. Fig. 1) des Kopfbereiches 110.

Der Schneidebereich der selbstschneidenden Schraube 100 umfasst denjenigen spitzenseitigen und mit einem Gewinde versehenen Bereich 106 der Schraube 100, der das Gegengewinde in das Knochenmaterial schneidet. Das ist der Bereich, in dem das Gewinde seinen größten Außendurchmesser erreicht und beibehält. Hierbei wird außer Acht gelassen, dass Außen- und/oder Kerndurchmesser sich ggf. im Kopfbereich 110 nochmals vergrößern. Der Schneidebereich 106 endet kopfseitig an der Stelle, an der sowohl der größte Außen- als auch der größte Kerndurchmesser erreicht sind und der Kerndurchmesser (oder der Außendurchmesser, oder beide Durchmesser) in Richtung Zwischenbereich 108 abnimmt.

Bei der Schraube 100 ist der Kerndurchmesser des Schneidebereichs 106 gegenüber demjenigen des Zwischenbereichs 108 vergrößert. Allgemein gilt, dass, wenn man nur den Kerndurchmesser betrachtet, der spitzenseitige Abschnitt der Schraube 100 zum Beispiel eine ballige Form aufweisen kann. Hierzu kann der Kerndurchmesser im Schneidebereich 106 etwa in Form einer konvexen Kurve variieren, während er im angrenzenden Teil des Zwischenbereiches (und unter Umständen auch der Spitze) konstant ist. Im Beispiel der Knochenschraube 100 ist der Kerndurchmesser im Bereich des Schneidebereiches 106 weniger ballig, sondern vielmehr konstant. Zwischen balliger Form und einem konstanten, erweitertem Kerndurchmesser sind Zwischenformen möglich, etwa ein mehrfach abgestufter Kerndurchmesser im Schneidebereich.

Ein Übergangsbereich 114 ist zwischen Schneidebereich 106 und Zwischenbereich 108 dadurch definiert, dass hier der Kerndurchmesser des Schneidebereichs 106 in den Kerndurchmesser des Zwischenbereichs 108 übergeht. Wie aus den Figuren ersichtlich, verfügt der Zwischenbereich 108 selbst über einen konstanten Kerndurchmesser. Der Außendurchmesser des Schraubenschaftes 102 erreicht, von der Spitze aus gesehen, im Schneidebereich 106 seinen größten Wert und ist im weiteren Verlauf im Schneidebereich 106 und Zwischenbereich 108 konstant.

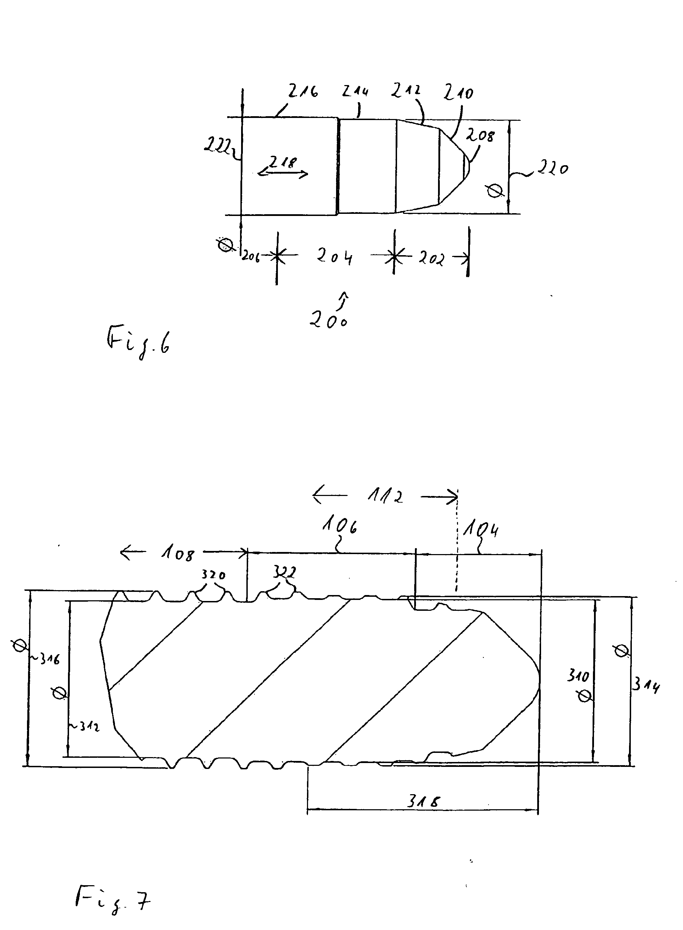

In der Fig. 6 ist schematisch ein Schraubenrohling 200 gezeigt, aus welcher die Schraube 100 herausgearbeitet wird. Aus einem Bereich 202 geht die Zentrierspitze 104 hervor, aus einem Bereich 204 der Schneidebereich 106 und aus einem Bereich 206 der Zwischenbereich 108. Der Bereich 202 umfasst das abgerundete Segment 208 sowie die beiden trapezförmigen Segmente oder Kalotten 210 und 212 mit jeweils unterschiedlichen Öffnungswinkeln, aus denen sich die abgestufte Form der späteren Zentrierspitze 104 ergibt. Das Segment 210 bleibt gewindefrei, der Gewindeabschnitt 112 beginnt am Segment 212. Weitere Segmente 214 und 216 sind zylindrisch ausgebildet. Der im Schneidebereich 106 gegenüber dem Zwischenbereich 108 verdickte Kerndurchmesser wird erst im Zuge des Gewindefräsens ausgebildet.

Bei anderen Ausführungen können statt der in Fig. 6 gezeigten abgestuften Spitze 202 auch kontinuierliche Formen verwendet werden. Generell wird die Spitze vorteilhaft als Zentrierspitze mit auslaufendem Gewinde vorgesehen.

Fig. 7 ist ein Querschnitt durch den in Fig. 4 gezeigten Teil der Knochenschraube 100 mit sich rechtwinklig öffnender Spitze 104, Schneidebereich 106 sowie dem spitzenseitigen Teil des Zwischenbereichs 108.

Der größte Kerndurchmesser 310 des Gewindebereichs 112 wird im Schneidebereich 106 erreicht. Über den Schneidebereich 106 ist der Kerndurchmesser konstant. Im Übergangsbereich 114 zwischen Schneidebereich 106 und Zwischenbereich 108 nimmt der Kerndurchmesser auf einen kleineren Wert 312 ab, der über den Zwischenbereich 108 beibehalten wird. Der Außendurchmesser nimmt von der Spitze 104 über einen Wert 314 am Übergang zum Schneidebereich 106 zu und erreicht seinen maximalen Wert 316 im Schneidebereich 106. Der Außendurchmesser ist im Übergangsbereich 114 und im Zwischenbereich 108 konstant mit dem Wert 316.

Wie aus der Fig. 7 zu ersehen, erfolgt bei der hier beispielhaft beschriebenen Knochenschraube 100 der Übergang vom vergrößerten Kerndurchmesser 310 im Schneidebereich 106 auf den kleineren Kerndurchmesser 312 im Zwischenbereich 108 abgestuft, das heißt, der Übergang erfolgt im Bereich 114 in Form einer einzigen Stufe. Bei anderen Ausführungen der Knochenschraube kann die Abstufung stattdessen in Form mehrerer Stufen vorgenommen werden, oder der Kerndurchmesser wird im Übergangsbereich beispielsweise in Form einer konvexen Kurve zurückgeführt. Auch Mischformen aus abgestufter und kontinuierlicher Verringerung des Kerndurchmessers sind möglich.

Bei dem in Fig. 7 gezeigten einstufigen Übergang vom konstanten Kerndurchmesser 310 im Schneidebereich 106 zum konstanten Kerndurchmesser 312 im Zwischenbereich 108 ist der Übergangsbereich 114 mit der Grenze zwischen Schneidebereich 106 und Zwischenbereich 108 identisch. Bei anderen Ausführungen, bei denen der Kerndurchmesser zwischen Schneide- und Zwischenbereich in mehreren Stufen und/oder kontinuierlich abnimmt, ist der Übergangsbereich entsprechend ausgedehnter.

In dem Beispiel der Knochenschraube 100 ist der Kerndurchmesser 312 im Zwischenbereich konstant. Generell ist es nicht zwingend erforderlich, dass der Kerndurchmesser im Zwischenbereich konstant ist. Allerdings sollte der Kerndurchmesser im Zwischenbereich kleiner als der Kerndurchmesser im Schneidebereich sein. Weiterhin ist bei dem in den Fign. 1-7 gezeigten Ausführungsbeispiel der Knochenschraube 100 der Kerndurchmesser 310 im Schneidebereich 106 konstant. Bei anderen Ausführungsbeispielen kann der Kerndurchmesser im Schneidebereich variieren, und kann etwa insgesamt oder stückweise eine konvexe Kurve beschreiben, beispielsweise beim Übergang von der Spitze zum Schneidebereich (in Fig. 7 erfolgt der Übergang des Kerndurchmessers von der Spitze 104 zum Schneidebereich 106 gestuft).

Da der Kerndurchmesser 310 im Schneidebereich 106 größer ist als der Kerndurchmesser im Zwischenbereich 108, wird durch den Schneidebereich 106 ein größeres Loch geschnitten als es dem Kerndurchmesser 312 im Zwischenbereich 108 entspricht. Dadurch wird im Zwischenbereich 108 bezogen auf den durchschnittenen Knochen die Auflagefläche der Schraube 100 und die Eindringtiefe der Gewindezähne 320 reduziert. Dies führt zu einer Reduktion der Eindrehkräfte der Schraube 100 in den Knochen. Damit die Schraube 100 nach dem Eindrehen im Zwischenbereich 108 nicht locker in dem durch den Schneidebereich 106 geschnittenen Gegengewinde liegt, sollte die geschnittene Geometrie korrespondieren. Das heißt, das Gewinde im Schneidebereich 106 und Zwischenbereich 108 sollte durchgehend und mit konstanter Gewindesteigung ausgeführt sein. Sind alternativ mehrere (durch gewindefreie Abschnitte getrennte) Gewindeabschnitte im Schneidebereich und im Zwischenbereich vorgesehen, sollten diese zueinander korrespondieren, d.h. die Gewinde sollten zueinander synchron sein.

Wie besonders in den Fign. 3, 4 und 7 erkennbar, ändert sich das Gewindeprofil beim Übergang vom Zwischenbereich 108 zum Schneidebereich 106. Während im Schneidebereich 106 (und im in der Spitze 104 auslaufenden Gewindeteil) das Gewinde trapezförmig mit verhältnismäßig großer Oberfläche der Zähne (Außenfläche der Schraube) ausgebildet ist, vgl. die Gewindezähne 322 in Fig. 7, entspricht die Gewindeform im Zwischenbereich 108 einem Trapezgewinde mit verhältnismäßig kleinerer Oberfläche der Zähne ("spitzeren" Gewindezähnen), vgl. die Gewindezähne 320. Bei anderen Ausführungsbeispielen kann im Zwischenbereich ein Spitzgewinde vorliegen. Hiervon unberührt bleibt, dass die Kanten der Schraube 100 generell gebrochen sind, so dass auch die Gewindezähne 320 in gewissem Umfang abgerundet sind.

Der trapezförmige Querschnitt der Gewindegänge im Schneidebereich 106 dient insbesondere zum Durchschneiden des Knochenmaterials beim Eindrehen, während die Schneidfunktion bei dem Spitzgewinde im Zwischenbereich 108 zurücktritt. Im Zwischenbereiche 108 soll sich das Gewinde insbesondere in die bereits geschnittenen Gewindegänge einfügen, ohne dass hierdurch der Eindrehwiderstand signifikant steigt.

Bei einem Verfahren zur Herstellung der Schraube 100 aus dem Schraubenrohling 200 kann ein Fräswerkzeug verwendet werden, um das Gewinde herzustellen. Hierbei kann das Fräswerkzeug etwa über eine konvexe Kurve oder eine Abstufung mit einer oder mehreren Stufen geführt werden. Dadurch werden die Gewindezähne im Schneidebereich 106 weniger tief geschnitten als im Zwischenbereich 108, so dass sich der vergrößerte Kerndurchmesser 310 im Schneidebereich 106 im Verhältnis zum Kerndurchmesser 312 des Zwischenbereiches 316 ergibt.

Nachfolgend werden Beispiele für konkrete Abmessungen der Knochenschraube 100 angegeben. Häufig werden gültige Wertebereiche mit jeweils einem unteren und einem oberen Wert angegeben; aus der Kombination der unteren Werte ergibt sich ein konkretes Ausführungsbeispiel einer kleineren Schraube, während die Kombination der oberen Werte ein konkretes Ausführungsbeispiel einer größeren Schraube ergibt. Es sind jedoch ohne weiteres auch Beispiele denkbar, die außerhalb der angegebenen Wertebereiche liegen; dem Fachmann sind die allgemeinen Abmessungen von Knochenschrauben, insbesondere Verriegelungsschrauben bekannt. Es kommt bei den hier angegebenen Zahlenwerten neben den absoluten Werten auch auf das Verhältnis der Werte der verschiedenen Bemaßungen zueinander an.

Allgemein gilt, dass ein typischer Flankendurchmesser der Knochenschraube 100 beispielsweise zwischen 2 Millimetern (mm) und 8,0 mm liegen kann, bevorzugt zwischen 2,7 mm und 5,0 mm; kleinere oder größere Flankendurchmesser sind ebenso möglich, jedoch beziehen sich die nachfolgenden Wertebereiche auf Schrauben mit den angegebenen Flankendurchmessern. Die Toleranz der beispielhaft angegebenen Bemaßungen liegt typischerweise im Bereich von 0,1 mm.

Die Verhältnisse für die Schraubenspitze 104 werden der erleichterten Erkennbarkeit aufgrund des fehlenden Gewindes wegen anhand des in Fig. 6 gezeigten Schraubenrohlings 200 erläutert. Das an das gerundete Spitzensegment 208 anschließende Segment 210 kann einen Kegelstumpf mit einen Öffnungswinkel von 90° bilden, das heißt, die Mantellinien des Kegelstumpfes bilden einen Winkel von 45° gegen die Schrauben(rohlings)achse 218. Spitzere oder stumpfere Öffnungswinkel sind ebenfalls möglich; jedoch sollten vorzugsweise die selbstzentrierenden Eigenschaften der Schraube erhalten bleiben. Der durch das anschließende Segment 212 gebildete Kegelstumpf kann beispielsweise einen Öffnungswinkel von 24° haben, d.h. die Mantellinien bilden einen Winkel von 12° gegen die Schraubenachse 218.

Das zylindrische Segment 214 kann einen Durchmesser 220 etwa im Bereich von 4,9 mm bis 2,8 mm haben. Der anschließende Rohlingsschaft 216 kann beispielsweise einen Durchmesser 222 von 5,1 mm bis 3,0 mm haben. Eine Länge der Segmente 208, 210, 212 und 214 entlang der Schraubenachse 218 kann beispielsweise im Bereich von 6,7 mm bis 4,5 mm liegen. Eine Länge nur der Segmente 208, 210 und 212 könnte sich dann beispielsweise zu 3,76 mm bis 2,59 mm ergeben, und eine Länge nur der Segmente 208 und 210 könnte sich zu 1,6 mm bis 0,55 mm ergeben. Bezogen auf Fig. 7 beträgt der Nenndurchmesser 314 am spitzenseitigen Teil des Schneidebereichs 106 beispielsweise 4,9 mm bis 2,8 mm, während der Nenndurchmesser 316 am kopfseitigen Teil des Schneidebereichs 106 beispielsweise 5,1 mm bis 3,0 mm beträgt. Der Kerndurchmesser 310 im Schneidebereich 106 kann beispielsweise einen Wert im Bereich von 4,7 mm bis 2,7 mm annehmen. Der Kerndurchmesser 312 im Zwischenbereich 108 hat demgegenüber einen kleineren Wert im Bereich von 4,5 mm bis 2,5 mm.

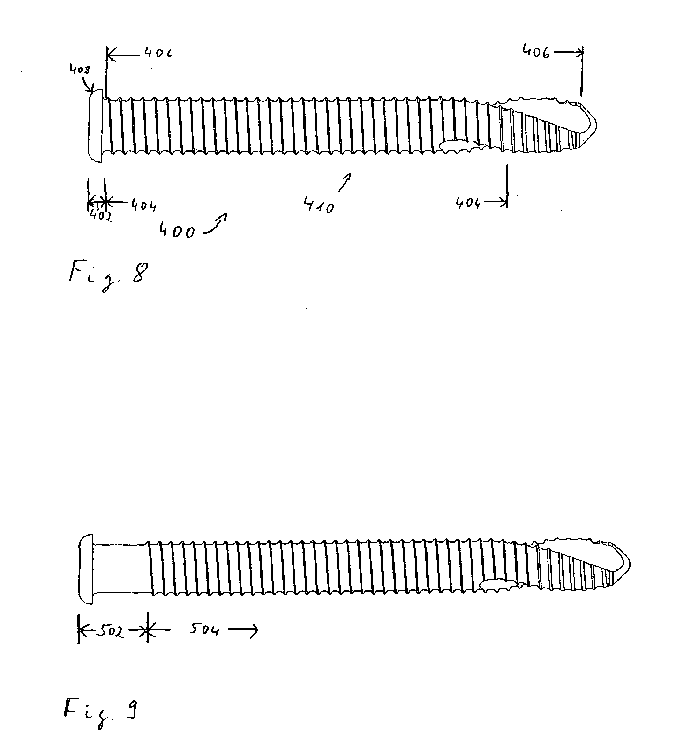

Eine Länge des Schneidebereichs 106 entlang der Schraubenachse 218 (Fig. 6) kann beispielsweise zwischen 4.7 mm und 2.0 mm liegen. Entsprechend kann eine Länge des Schneidebereichs 104 zwischen 3,5 mm bis 2,7 mm liegen. Die Länge 318 in Fig. 8 gibt den Abstand von der Spitze der Schraube bis zu dem Punkt an, ab dem der maximale Nenn- bzw. Außendurchmesser der Schraube 100 erreicht wird. Die Länge 318 kann beispielsweise einen Wert zwischen 6,7 mm bis 4,5 mm haben.

Der Flankenwinkel des Trapezgewindes im Schneidebereich 106 kann beispielsweise 45° betragen.

Die insbesondere in Fig. 4 gezeigte Nute 116 kann auf einen Außendurchmesser von 4,7 mm bis 2,0 mm auslaufend ausgebildet sein. Die Nutensteigung der Nute 116 und 118 kann typischerweise 40 mm betragen. Die Länge 124 der Nut entlang der Schraubenachse 126 (vgl. Figuren 2 und 4) kann zwischen 20 mm bis 2 mm und bevorzugt zwischen 12,5 mm bis 6,0 mm betragen. Allerdings kann die Länge 124 der Nute 116 nahezu unabhängig von der Gesamtlänge der Schraube im wesentlichen konstant zu 12,5 mm gewählt werden. Lediglich bei besonders kurzen Schrauben, beispielsweise mit einer Länge von weniger als 20 mm, kann eine kürzere Länge 124 der Nut entlang der Schraubenachse, bspw. mit einem Wert von 6,2 mm vorgesehen sein.

Die Kanten der Nut 116 und 118 sind scharf aber gratfrei ausgeführt. Die Nute 116 und 118 sind um 180° mit einer Toleranz von zum Beispiel 1° versetzt. Bezogen auf Fig. 5 haben die Nute 116 und 118 an der Spitze 104 einen Abstand 128 von 2 mm, wenigstens aber 0,8 mm.

Fig. 8 zeigt ein weiteres Ausführungsbeispiel einer Knochenschraube 400. Diese unterscheidet sich von der Knochenschraube 100 der vorhergehenden Figuren in der Ausführung des Kopfbereichs. Während bei dem Kopfbereich 110 der Schraube 100 (vgl. Fig. 1) sowohl der Kerndurchmesser als auch der Außendurchmesser im Vergleich zu den entsprechenden Durchmessern des Zwischenbereiches 108 vergrößert ist, weist der Kopfbereich 402 der Schraube 400 sowohl den gleichen Kerndurchmesser als auch den gleichen Außendurchmesser wie der Zwischenbereich 404 auf. Da der zum Eindrehen und ggf. Verriegeln nutzbare Gewindeabschnitt 406 sich bei der Schraube 400 bis unmittelbar an das Kopfteil 408 erstreckt, kann der Schraubenschaft 410 der Schraube 400 insgesamt kürzer ausgeführt sein als der Schraubenschaft 102 der Schraube 100.

Fig. 9 zeigt eine weitere Ausführung 500 einer Knochenschraube, bei dem der Kopfbereich 502 nochmals anders gestaltet ist als bei den Schrauben 100 und 400. Der Kopfbereich 502 ist insbesondere gewindefrei ausgeführt. Der Durchmesser des Kopfbereiches 502 entspricht dem Kerndurchmesser des Zwischenbereiches 504.

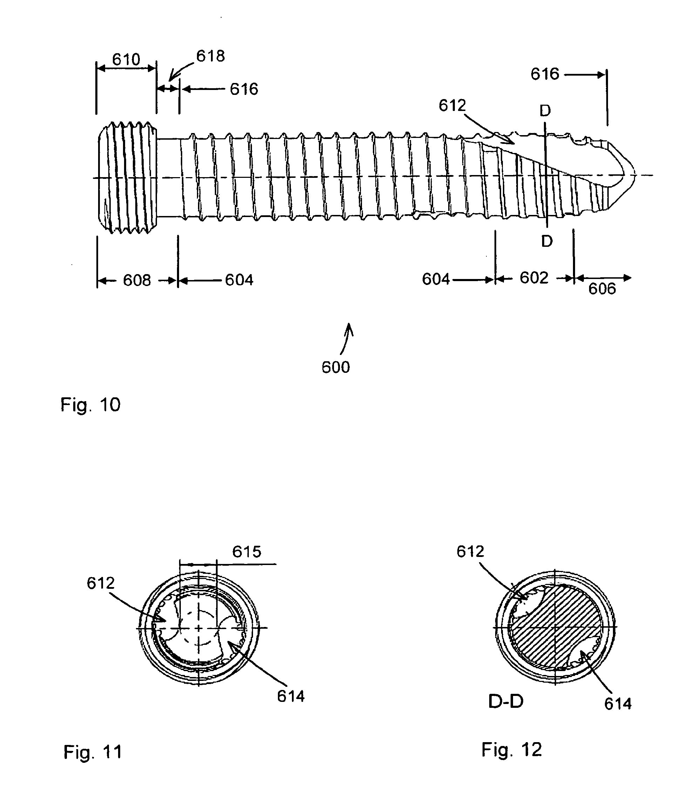

Die Fig. 10 zeigt ein weiteres Ausführungsbeispiel einer Knochenschraube 600. Wie die vorhergehenden Ausführungsbeispiele auch weist die Schraube 600 im Schneidebereich 602 einen vergrößerten Kerndurchmesser im Vergleich zum Zwischenbereich 604 auf. Der Außendurchmesser der Schraube ist im Schneidebereich und im Zwischenbereich konstant. Die stumpfe Schraubenspitze 606 hat einen Öffnungswinkel von 90°. Ein Kopfbereich 608 hat einen erweiterten Kopfteil 610 mit eigenem Gewinde.

Fig. 11 zeigt die Schraube 600 von vorn und Fig. 12 zeigt einen Schnitt durch die Schraube 600 entlang der Linie D - D im Schneidebereich 602 . Erkennbar sind zwei sich verjüngende Nuten 612, 614, die in gleicher Weise ausgebildet sein können wie für die Nute 116, 118 der Schraube 100 beschrieben. Die Nute 612 und 614 sind um 180° versetzt. Ferner ist das Gewinde des Kopfteils 610 zu erkennen.

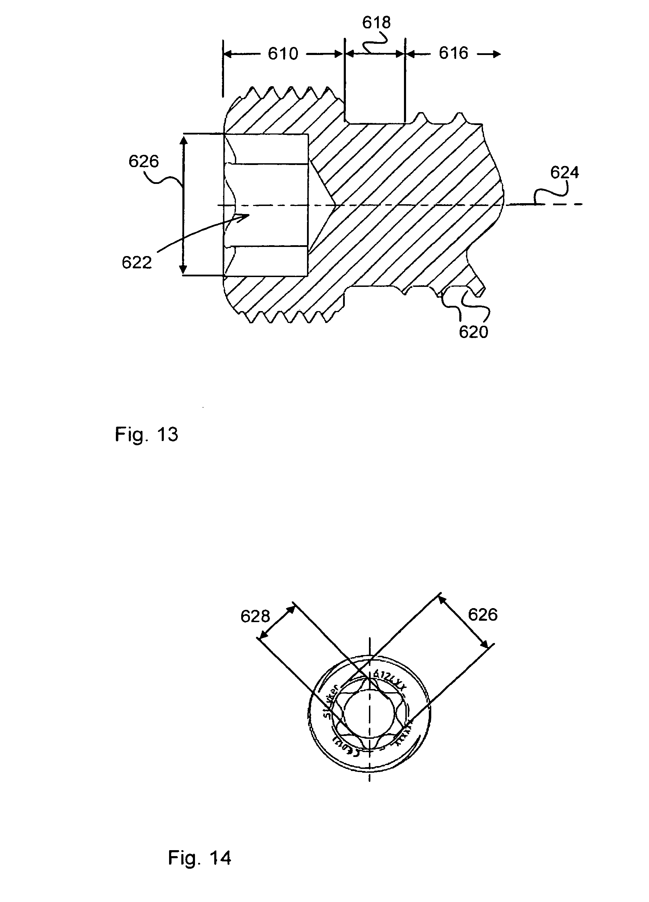

Fig. 13 ist eine Schnittansicht des hinteren Teils der Schraube 600. Wie aus den beiden Figuren 10 und 13 ersichtlich, endet der vordere Gewindeabschnitt 616 im Zwischenbereich 604 am Kopfbereich 608, der somit einen gewindefreien Abschnitt 618 hat. Der Kopfteil 610 hat einen gegenüber dem Zwischenbereich 604 deutlich vergrößerten Durchmesser, das heißt, sowohl der Kern- als auch der Außendurchmesser des mit Gewinde versehenen Kopfteils 610 ist größer als der entsprechende Durchmesser des Zwischenbereiches 604. Die Gewinde im Gewindeabschnitt 616 und im Kopfteil 610 laufen zueinander synchron. Bei dem Gewinde im Kopfteil 610 kann es sich um ein zweigängiges Gewinde handeln.

Wie für das Beispiel der Knochenschraube 100 ausgeführt, können auch bei der Schraube 600 (vgl. Fig. 13) die Gewindezähne 620 als schmale Trapeze ausgebildet sein (insbesondere im Vergleich zu den Gewindezähnen im Schneidebereich), so dass sich ein "spitzes" Trapezgewinde ergibt. Alternativ kann das Gewinde im Zwischenbereich als Spitzgewinde ausgeführt sein, wobei die Gewindezähne gebrochen sein können.

Der Kopfbereich 608 ist mit einer Aussparung 622 zur Aufnahme eines Schraubenschlüssels ausgebildet, beispielsweise als Innensechsrund. Fig. 14 ist eine Aufsicht auf das hintere Ende der Schraube 600 mit Kopfbereich 608 und Aussparung 622.

Eine Gesamtlänge der Schraube 600 kann beispielsweise zwischen 8 mm und 150 mm, bevorzugt zwischen 14 mm und 120 mm liegen. Die Länge des Kopfteils 612 entlang der Schraubenachse 624 kann beispielsweise 3,2 mm betragen. Der gewindefreie Abschnitt 618 kann eine Länge von 1,3 mm haben. Bei einem Flankendurchmesser der Schraube 600 von 4,7 mm im Zwischenbereich 604 kann der Kerndurchmesser 4,5 mm im Zwischenbereich 604 (4,7 mm im Schneidebereich 602) betragen und der Außendurchmesser kann 5,1 mm im Zwischenbereich 604 und Schneidebereich 602 betragen. Der verdickte Kopfteil 610 kann dabei einen Kerndurchmesser von 5,8 mm und einen Außendurchmesser von 6,5 mm haben.

Die Gewindezähne 620 können einen Abstand von 1 mm zwischen den Zähnen und eine obere Trapezfläche von 0,1 mm haben. Der Abstand zwischen den Grundflächen zweier Gewindezähne am Schraubenschaft kann ungefähr 0,323 mm betragen.

Der äußere Durchmesser 626 des Innensechsrunds 622 kann bspw. 3,95 mm betragen und der innerste Durchmesser 628 des Innensechsrunds 622 kann 2,85 mm betragen, jeweils mit einer Toleranz von einigen hundertstel Millimetern.

Alle der hier beschriebenen Knochenschrauben können als Verriegelungsschrauben für ein Implantat verwendet werden. Während der vordere Teil bei den Knochenschrauben 100, 400, 500 und 600 insbesondere in Bezug auf den im Schneidebereich vergrößerten Kerndurchmesser jeweils in gleicher Weise ausgebildet ist, sind die Schrauben mittels ihres Kopfbereiches an jeweils unterschiedliche Einsatzzwecke angepasst, bspw. an unterschiedliche Fixationselemente. Als Material für die hier beispielhaft dargestellten Knochenschrauben kann Edelstahl oder Titan verwendet werden.

Die vorstehend diskutierten Knochenschrauben sind mit einer gegenüber herkömmlichen Schrauben reduzierten Eindrehkraft in Knochen oder Knochenfragmente einschraubbar. Der im Schneidebereich der Knochenschraube vergrößerte Kerndurchmesser (im Vergleich zum Kerndurchmesser des Zwischenbereiches) bewirkt, dass die Auflagefläche der Schraube im Zwischenbereich reduziert wird, und gleichzeitig kann die Eindringtiefe der Gewindezähne reduziert werden. Ein vom Schneidebereich bis zum Zwischenbereich und gegebenenfalls zum Kopfbereich durchgängig durchgeführter Gewindeabschnitt (oder mehrere separate, synchrone Gewindeabschnitte) stellen sicher, dass die geschnittene Geometrie korrespondiert. Somit fügt sich der hinter dem Schneidebereich befindliche Zwischenbereiche der Schraube beim Eindrehen passgenau in das geschnittene Gegengewinde im Knochen ein. Hierbei wird vorteilhaft eine konstante Gewindesteigung vorgesehen.

Um den sicheren Sitz der Schraube auch im Zwischenbereich zu gewährleisten, kann die Gewindeform zwischen Schneidebereich und Zwischenbereich variieren, beispielsweise von einem Trapez- zu einem Spitzgewinde, oder von einem stumpferen Trapezgewinde zu einem spitzeren Trapezgewinde. Andere Gewindeformen sind ebenfalls denkbar, sofern sie die Funktionalität einer selbstschneidenden Schraube gewährleisten. Der konstante Außendurchmesser im Übergangsbereich, und vorzugsweise auch entlang zumindest eines wesentlichen Teils des Zwischenbereichs stellt hierbei den optimalen Sitz der Schraube sicher. Ein sich durchgehend vom Schneidebereich über den gesamten Zwischenbereich erstreckendes Gewinde verbessert dabei den passgenauen Sitz weiter, ohne dass sich die Eindrehkräfte wesentlich erhöhen. Eine oder mehrere Nute können vorgesehen werden, um Schnittgut abzuführen, ohne dass die Funktionalität der Schraube in Bezug auf die Reduktion der Eindrehkräfte bei sicherem Sitz dadurch beeinträchtigt wird.

Je nach konkretem Einsatzzweck kann es zweckmäßig sein, im Kopfbereich einen in Bezug auf Kerndurchmesser und/oder Außendurchmesser vergrößerten Kopfteil vorzusehen, um einen sicheren Sitz der Knochenschraube im Knochenmaterial und/oder einem weiteren Implantatelement zu gewährleisten. Der entsprechend verdickte Kopfteil kann ein eigenes, gegebenenfalls synchrones Gewinde haben, oder ein durchgehender Gewindeabschnitt erstreckt sich vom Zwischenbereich bis in den Kopfbereich hinein.

Die hier dargestellten Ausführungsbeispiele stellen nur einige zweckmäßige Ausführungsformen der Erfindung dar. Darüber hinaus sind im Geltungsbereich der Erfindung, der ausschließlich durch die nachfolgenden Ansprüche angegeben wird, durch fachmännisches Handeln noch viele weitere Ausführungsformen denkbar.

## Patentansprüche

1. Selbstschneidende Knochenschraube (100, 400, 500, 600) mit einem Schraubenschaft (102), der eine vordere Spitze (104), einen Schneidebereich (106), einen Zwischenbereich (108) und einen hinteren Kopfbereich (110) aufweist, wobei sich in einem Gewindeabschnitt (112) des Schraubenschaftes ein Gewinde wenigstens über einen Übergangsbereich (114) erstreckt, der aneinander angrenzende Teile des Schneidebereichs und des Zwischenbereichs umfasst, und durch das Gewinde im Gewindebereich ein Außendurchmesser und ein Kerndurchmesser des Schraubenschaftes definiert sind,
wobei im Übergangsbereich (114)
- der Kerndurchmesser des Schraubenschaftes im Schneidebereich größer ist als der Kerndurchmesser des Schraubenschaftes im Zwischenbereich,
**dadurch gekennzeichnet, daß** im Übergangsbereich
- der Außendurchmesser des Schraubenschaftes konstant ist.

2. Knochenschraube nach Anspruch 1,
**dadurch gekennzeichnet, dass**
der Kerndurchmesser am Übergang (114) vom Schneidebereich (106) zum Zwischenbereich (108) abgestuft ist.

3. Knochenschraube nach Anspruch 1 oder 2,
**dadurch gekennzeichnet, dass**
der Kern des Schneidebereichs (106) konvexe Form hat.

4. Knochenschraube nach einem der vorhergehenden Ansprüche,
**dadurch gekennzeichnet, dass**
der Kerndurchmesser im Zwischenbereich (108) konstant ist.

5. Knochenschraube nach einem der vorhergehenden Ansprüche,
**dadurch gekennzeichnet, dass**
der Außendurchmesser im Zwischenbereich (108) konstant ist.

6. Knochenschraube nach einem der vorhergehenden Ansprüche,
**dadurch gekennzeichnet, dass**
das Gewinde sich durchgehend über den Schneidebereich (106) und den Zwischenbereich (108) erstreckt.

7. Knochenschraube nach einem der vorhergehenden Ansprüche,
**dadurch gekennzeichnet, dass**
das Gewinde im Schneidebereich als Trapezgewinde (322) ausgeführt ist.

8. Knochenschraube nach einem der vorhergehenden Ansprüche,
**dadurch gekennzeichnet, dass**
das Gewinde eine konstante Gewindesteigung hat.

9. Knochenschraube nach einem der vorhergehenden Ansprüche,
**dadurch gekennzeichnet, dass**
das Gewinde in der Spitze (104) ausläuft.

10. Knochenschraube nach einem der vorhergehenden Ansprüche,
**dadurch gekennzeichnet, dass**
die Spitze (104) als Zentrierspitze ausgeführt ist.

11. Knochenschraube nach einem der vorhergehenden Ansprüche, **gekennzeichnet durch**
mindestens eine sich wenigstens über die Spitze (104) und den Schneidebereich (106) erstreckende Nut (116, 118) zum Wegführen von Schnittgut.

12. Knochenschraube nach einem der vorhergehenden Ansprüche,
**dadurch gekennzeichnet, dass**
der Kopfbereich (110, 608) ein Gewinde aufweist.

13. Knochenschraube nach einem der vorhergehenden Ansprüche,
**dadurch gekennzeichnet, dass**
der Außendurchmesser des Kopfbereiches (110, 608) größer ist als der Außendurchmesser des Zwischenbereiches (108, 604).

14. Knochenschraube nach Anspruch 13,
**dadurch gekennzeichnet, dass**
der Außendurchmesser des Kopfbereichs (110, 502, 608) im Bereich eines Kopfteils (122, 610) größer ist als der Außendurchmesser des Zwischenbereiches (108, 504, 604).

15. Knochenschraube nach Anspruch 14,
**dadurch gekennzeichnet, dass**
der Kopfteil (608) ein Gewinde aufweist.

16. Knochenschraube nach Anspruch 14 oder 15,
**dadurch gekennzeichnet, dass**
der Kopfbereich (502, 610) in einem vom Kopfteil (608) verschiedenen Bereich einen gewindefreien Abschnitt (618) aufweist.

17. Knochenschraube nach Anspruch 16,
**dadurch gekennzeichnet, dass**
der gewindefreie Abschnitt (618) den gleichen Kerndurchmesser wie der Zwischenbereich (504, 604) aufweist.

18. Knochenschraube nach einem der vorhergehenden Ansprüche zur Anwendung als Verriegelungsschraube für ein Implantat.

19. Verfahren zur Herstellung einer selbstschneidenden Knochenschraube (100, 400, 500, 600) mit einem Schraubenschaft (102), der eine vordere Spitze (104), einen Schneidebereich (106), einen Zwischenbereich (108) und einen hinteren Kopfbereich (110) aufweist, wobei sich in einem Gewindeabschnitt (112) des Schraubenschaftes ein Gewinde über einen Übergangsbereich (114) erstreckt, der aneinander angrenzende Teile wenigstens des Schneidebereichs und des Zwischenbereichs umfasst, und wobei durch das Gewinde im Gewindebereich ein Außendurchmesser und ein Kerndurchmesser des Schraubenschaftes definiert sind,
mit dem folgenden Schritt:
Führen eines Fräswerkzeuges zur Herstellung des Gewindes im Gewindeabschnitt (112) derart, dass die Gewindezähne im Schneidebereich (106) weniger tief geschnitten werden als im Zwischenbereich (108),
so dass im Übergangsbereich
- der Kerndurchmesser des Schraubenschaftes im Schneidebereich größer ist als der Kerndurchmesser des Schraubenschaftes im Zwischenbereich, und
- der Außendurchmesser des Schraubenschaftes konstant ist.

## Claims

1. A self-tapping bone screw (100, 400, 500, 600) having a screw shank (102), which has a front tip (104), a cutting region (106), an intermediate region (108) and a rear head region (110), wherein a thread extends, in a threaded portion (112) of the screw shank, over at least a transition region (114) comprising mutually adjoining parts of the cutting region and the intermediate region, and an outside diameter and a root diameter of the screw shank being defined by the thread in the threaded region,
wherein in the transition region (114)
- the root diameter of the screw shank in the cutting region is greater than the root diameter of the screw shank in the intermediate region,
**characterized in that** in the transition region
- the outside diameter of the screw shank is constant.

2. The bone screw according to claim 1,
**characterized in that**
the root diameter at the transition (114) from the cutting region (106) to the intermediate region (108) is stepped.

3. The bone screw according to claim 1 or 2,
**characterized in that**
the root of the cutting region (106) has a convex shape.

4. The bone screw according to one of the preceding claims,
**characterized in that**
the root diameter in the intermediate region (108) is constant.

5. The bone screw according to one of the preceding claims,
**characterized in that**
the outside diameter in the intermediate region (108) is constant.

6. The bone screw according to one of the preceding claims,
**characterized in that**
the thread extends continuously over the cutting region (106) and the intermediate region (108).

7. The bone screw according to one of the preceding claims,
**characterized in that**
the thread in the cutting region is designed as a trapezoidal thread (322).

8. The bone screw according to one of the preceding claims,
**characterized in that**
the thread has a constant thread pitch.

9. The bone screw according to one of the preceding claims,
**characterized in that**
the thread runs out in the tip (104).

10. The bone screw according to one of the preceding claims,
**characterized in that**
the tip (104) is designed as a centring tip.

11. The bone screw according to one of the preceding claims,
**characterized by**
at least one groove (116, 118), extending over at least the tip (104) and the cutting region (106), for removing cut material.

12. The bone screw according to one of the preceding claims,
**characterized in that**
the head region (110, 608) has a thread.

13. The bone screw according to one of the preceding claims,
**characterized in that**
the outside diameter of the head region (110, 608) is greater than the outside diameter of the intermediate region (108, 604).

14. The bone screw according to claim 13,
**characterized in that**
the outside diameter of the head region (110, 502, 608) is, in the area of a head part (122, 610), greater than the outside diameter of the intermediate region (108, 504, 604).

15. The bone screw according to claim 14,
**characterized in that**
the head part (608) has a thread.

16. The bone screw according to claim 14 or 15,
**characterized in that**
the head region (502, 610) has, in an area different from the head part (608), a thread-free portion (618).

17. The bone screw according to claim 16,
**characterized in that**
the thread-free portion (618) has the same root diameter as the intermediate region (504, 604).

18. The bone screw according to one of the preceding claims for use as a locking screw for an implant.

19. A method for manufacturing a self-tapping bone screw (100, 400, 500, 600) having a screw shank (102), which has a front tip (104), a cutting region (106), an intermediate region (108) and a rear head region (110), wherein a thread extends, in a threaded portion (112) of the screw shank, over a transition region (114) comprising mutually adjoining parts of at least the cutting region and the intermediate region, and wherein an outside diameter and a root diameter of the screw shank are defined by the thread in the threaded region,
having the following step:
guiding a milling tool for producing the thread in the threaded portion (112) in such a way that the thread teeth are cut less deeply in the cutting region (106) than in the intermediate region (108),
so that in the transition region
- the root diameter of the screw shank in the cutting region is greater than the root diameter of the screw shank in the intermediate region, and
- the outside diameter of the screw shank is constant.

## Revendications

1. Vis à os autocoupante (100, 400, 500, 600) comportant une tige de vis (102) qui présente une pointe avant (104), une zone de coupe (106), une zone intermédiaire (108) et une zone de tête arrière (110), un filetage s'étendant le long d'un tronçon fileté (112) de la tige de vis au moins sur une zone de transition (114) qui comprend des parties adjacentes les unes des autres de la zone de coupe et de la zone intermédiaire, et le filetage définissant dans la zone filetée un diamètre extérieur et un diamètre du noyau de la tige de vis,
- le diamètre du noyau de la tige de vis dans la zone de transition (114) étant, dans la zone de coupe, supérieur au diamètre du noyau de la tige de vis dans la zone intermédiaire,
**caractérisée en ce que**
- le diamètre extérieur de la tige de vis est constant dans la zone de transition (114).

2. Vis à os selon la revendication 1,
**caractérisée en ce que**
le diamètre du noyau de la tige de vis est, à la zone de transition (114), étagé de la zone de coupe (106) à la zone intermédiaire (108).

3. Vis à os selon la revendication 1 ou 2,
**caractérisée en ce que**
le noyau de la zone de coupe (106) possède une forme convexe.

4. Vis à os selon l'une des revendications précédentes,
**caractérisée en ce que**
le diamètre du noyau est constant dans la zone intermédiaire (108).

5. Vis à os selon l'une des revendications précédentes,
**caractérisée en ce que**
le diamètre extérieur est constant dans la zone intermédiaire (108).

6. Vis à os selon l'une des revendications précédentes,
**caractérisée en ce que**
le filetage s'étend sans interruption sur la zone de coupe (106) et la zone intermédiaire (108).

7. Vis à os selon l'une des revendications précédentes,
**caractérisée en ce que**
le filetage est réalisé dans la zone de coupe sous forme de filet trapézoïdal (322).

8. Vis à os selon l'une des revendications précédentes,
**caractérisée en ce que**
le filetage possède un pas de vis constant.

9. Vis à os selon l'une des revendications précédentes,
**caractérisée en ce que**
le filetage débouche dans la pointe (104).

10. Vis à os selon l'une des revendications précédentes,
**caractérisée en ce que**
la pointe (104) est réalisée sous forme de pointe de centrage.

11. Vis à os selon l'une des revendications précédentes,
**caractérisée par**
au moins une rainure (116, 118) s'étendant au moins sur la pointe (104) et sur la zone de coupe (106) pour évacuer la matière coupée.

12. Vis à os selon l'une des revendications précédentes,
**caractérisée en ce que**
la zone de tête (110, 608) présente un filetage.

13. Vis à os selon l'une des revendications précédentes,
**caractérisée en ce que**
le diamètre extérieur de la zone de tête (110, 608) est supérieur au diamètre extérieur de la zone intermédiaire (108, 604).

14. Vis à os selon la revendication 13,
**caractérisée en ce que**
le diamètre extérieur de la zone de tête (110, 502, 608) est, dans la zone d'une partie de tête (122, 610), supérieur au diamètre extérieur de la zone intermédiaire (108, 504, 604).

15. Vis à os selon la revendication 14,
**caractérisée en ce que**
la partie de tête (608) présente un filetage.

16. Vis à os selon la revendication 14 ou 15,
**caractérisée en ce que**
la partie de tête (502, 610) présente dans une zone différente de la partie de tête (608) un tronçon (618) sans filetage.

17. Vis à os selon la revendication 16,
**caractérisée en ce que**
le tronçon (618) sans filetage présente le même diamètre du noyau que la zone intermédiaire (504, 604).

18. Vis à os selon l'une des revendications précédentes, destinée à servir de vis de verrouillage pour un implant.

19. Procédé pour la fabrication d'une vis à os autocoupante (100, 400, 500, 600) comportant une tige de vis (102) qui présente une pointe avant (104), une zone de coupe (106), une zone intermédiaire (108) et une zone de tête arrière (110), un filetage s'étendant le long d'un tronçon fileté (112) de la tige de vis au moins sur une zone de transition (114) qui comprend des parties adjacentes les unes des autres de la zone de coupe et de la zone intermédiaire, et le filetage définissant dans la zone filetée un diamètre extérieur et un diamètre du noyau de la tige de vis,
comprenant l'étape suivante :
conduite d'un outil de fraisage pour la réalisation d'un filetage le long du tronçon fileté (112) de telle sorte que la profondeur de coupe des dents du filet est moins importante dans la zone de coupe (106) que dans la zone intermédiaire (108),
si bien que, dans la zone de transition,
- le diamètre du noyau de la tige de vis est, dans la zone de coupe, supérieur au diamètre du noyau de la tige de vis dans la zone intermédiaire, et
- le diamètre extérieur de la tige de vis est constant.
